# EUROPEAN PATENT APPLICATION

(11) **EP 4 563 065 A1**
(43) Date of publication of application: **04.06.2025**
(21) Application number: 23846144.6
(22) Date of filing: 03.07.2023
(51) Int. Cl.: A61B 1/00, A61B 1/045

(54) **IMAGE CORRECTION METHOD IN ELECTRON ENDOSCOPE SYSTEM**

(30) Priority: 28.07.2022 JP 2022120393
(71) Applicant: HOYA CORPORATION, Shinjuku-ku Tokyo 160-8347 (JP)
(72) Inventor: MAKINO, Takao, Tokyo 160-8347 (JP)
(74) Representative: Schaumburg und Partner Patentanwälte mbB
(86) International application number: PCT/JP2023/024657
(87) International publication number: WO 2024/024411

(57) **Abstract**

One aspect of the present invention is a correction method of correcting a captured image in an electronic endoscope system. The method includes: a step of calculating a first correction parameter corresponding to a correction target processor using a reference endoscope and a reference processor; a step of causing the correction target processor to store the calculated first correction parameter; a step of calculating a second correction parameter corresponding to a correction target endoscope using the reference endoscope and the reference processor; a step of causing the correction target endoscope to store the calculated second correction parameter; and a step of performing, by the correction target processor, correction on a captured image captured by an image sensor of the correction target endoscope using the first correction parameter and the second correction parameter when the correction target endoscope and the correction target processor are connected.

## Description

### Technical Field

The present invention relates to an image correction method in an electronic endoscope system configured to acquire and process a captured image of a living tissue.

### Background Art

An electronic endoscope system is used to observe or treat a living tissue inside a human body. The electronic endoscope system includes an electronic endoscope (electronic scope) that captures an image of a living tissue with an image sensor and transmits the captured image to a processor, and the processor (processor for an electronic endoscope) that emits illumination light and processes a signal of the captured image to create an image for display.

Since there is an individual difference in spectral characteristics between an image sensor provided in the electronic endoscope (electronic scope) and a light source provided in the processor, when the electronic scope and the processor are connected, variance occurs in color information of the image of the living tissue to be displayed. It has been conventionally proposed to correct such an error (machine difference) in the color information between individuals of a processor and an endoscope.

For example, JP 5544219 B2 describes creation of a chromaticity correction table in order to correct a machine difference between a processor and an endoscope. The chromaticity correction table is for subjecting an image signal to correction of chromaticity corresponding to a difference between a spectral characteristic of standard light determined in advance as illumination light in the processor and a spectral characteristic of actual illumination light, and to correction of adjusting an imaging signal to a predetermined color tone serving as a reference according to a spectral characteristic of a color filter included in an image sensor of the endoscope.

### Summary of Invention

### Technical Problem

However, in a correction method described in JP 5544219 B2, in order to create a chromaticity correction table, a large amount of arithmetic operations of decimal numbers (for example, floating point arithmetic operations) are required in order to calculate a conversion coefficient for converting each of a plurality of spectral characteristics into a spectral characteristic serving as a reference and to obtain a product of conversion coefficients, and a large capacity memory is required to store data of each spectral characteristic. That is, there is a problem in the correction method described in JP 5544219 B2 that a processing load is enormous when correcting an error in color information between individuals of a processor and an endoscope.

Therefore, an object of the present invention is to reduce the processing load when correcting the color information in a case where arbitrary electronic endoscope and processor for an electronic endoscope are used in combination.

### Solution to Problem

One aspect of the present invention is a correction method of correcting a captured image in an electronic endoscope system to which an electronic endoscope that captures an image of a living tissue with an image sensor and a processor for an electronic endoscope that performs signal processing on a captured image by the image sensor to create a display image are connected.

The correction method includes:
a step of calculating a first correction parameter corresponding to a correction target processor which is a processor for an electronic endoscope to be corrected, using a reference endoscope which is an electronic endoscope serving as a reference for correction and a reference processor which is a processor for an electronic endoscope serving as a reference for correction;
a step of causing the correction target processor to store the calculated first correction parameter;
a step of calculating a second correction parameter corresponding to a correction target endoscope which is an electronic endoscope to be corrected, using the reference endoscope and the reference processor;
a step of causing the correction target endoscope to store the calculated second correction parameter; and
a step of performing, by the correction target processor, correction on a captured image captured by an image sensor of the correction target endoscope using the first correction parameter and the second correction parameter when the correction target endoscope and the correction target processor are connected.

The step of calculating the first correction parameter may include,
based on a first pixel value which is a pixel value of a captured image obtained when the reference endoscope and the correction target processor are connected and a predetermined color index is captured, and
based on a second pixel value which is a pixel value of a captured image obtained when the reference endoscope and the reference processor are connected and the color index is captured,
calculating, as the first correction parameter, a parameter for converting the first pixel value into the second pixel value.

The step of calculating the second correction parameter may include,
based on a third pixel value which is a pixel value of a captured image obtained when the correction target endoscope and the reference processor are connected and a predetermined color index is captured, and
based on a fourth pixel value which is a pixel value of a captured image obtained when the reference endoscope and the reference processor are connected and the color index is captured,
calculating, as the second correction parameter, a parameter for converting the third pixel value into the fourth pixel value.

The correction method may further include:
a step of specifying a first feature amount which is a feature amount obtained from the first pixel value and a second feature amount which is a feature amount obtained from the second pixel value, and calculating a first feature amount correction parameter for converting the first feature amount into the second feature amount;
a step of specifying a third feature amount which is a feature amount obtained from the third pixel value and a fourth feature amount which is a feature amount obtained from the fourth pixel value, and calculating a second feature amount correction parameter for converting the third feature amount into the fourth feature amount; and
a step of performing, by the correction target processor, correction on a feature amount obtained from a pixel value of a captured image captured by an image sensor of the correction target endoscope, using the first feature amount correction parameter and the second feature amount correction parameter when the correction target endoscope and the correction target processor are connected.

The color index may be determined from among a plurality of color indexes according to a feature amount obtained from a pixel value of a captured image.

### Advantageous Effects of Invention

According to the endoscope system described above, the processing load when correcting the color information in a case where arbitrary electronic endoscope and processor for an electronic endoscope are used in combination, can be reduced.

### Brief Description of Drawings

**Fig.** 1 is a diagram illustrating a schematic system configuration of an endoscope system according to an embodiment.
Fig. 2 is a block diagram illustrating a main configuration of the endoscope system according to the embodiment.
Fig. 3 is a diagram indicating each step of an image correction method in the electronic endoscope system according to the embodiment.
Fig. 4 is a diagram for describing a step of calculating a processor matrix in the electronic endoscope system according to the embodiment.
Fig. 5 is a diagram for describing a step of calculating a scope matrix in the electronic endoscope system according to the embodiment.
Fig. 6 is a flowchart indicating image processing at the time of diagnosis in the electronic endoscope system according to the embodiment.
Fig. 7 is a diagram for describing a step of calculating a processor matrix in an electronic endoscope system according to another embodiment.
Fig. 8 is a diagram for describing a step of calculating a scope matrix in the electronic endoscope system according to the another embodiment.

### (1) First Embodiment

Hereinafter, an electronic endoscope system according to an embodiment and an image correction method in the electronic endoscope system will be described in detail with reference to the drawings.

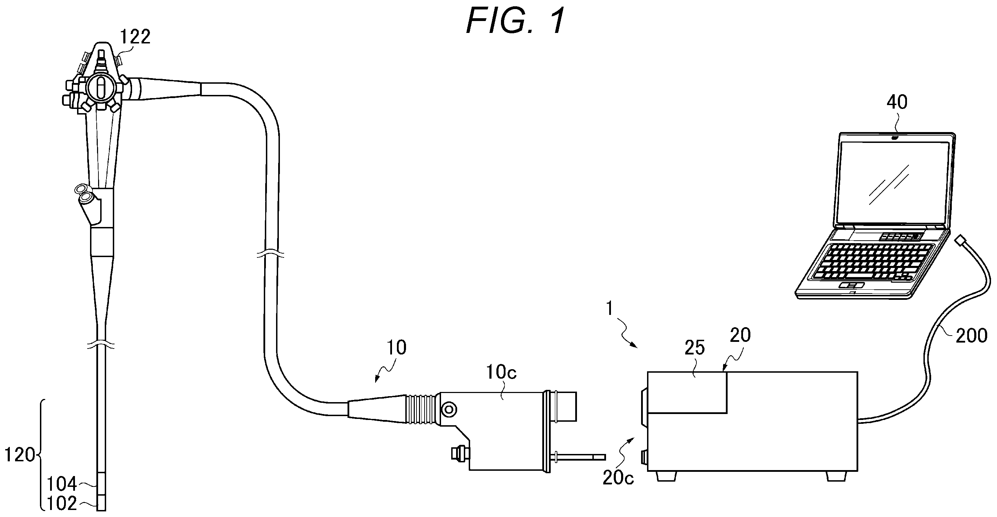

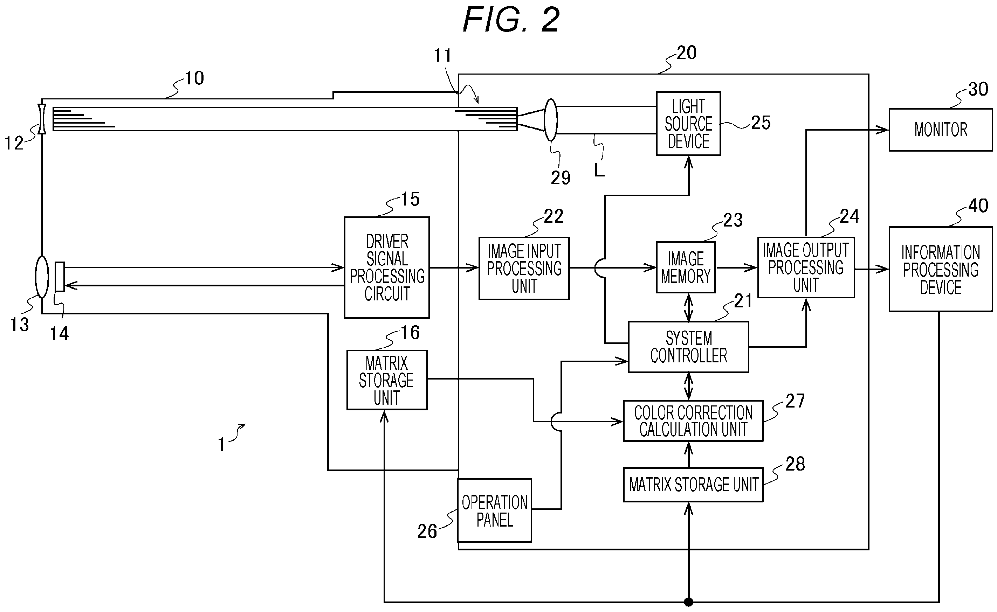

First, an electronic endoscope system 1 according to the embodiment will be described with reference to Figs. 1 and 2. Fig. 1 is a diagram illustrating a schematic system configuration of the endoscope system 1 according to the embodiment. Fig. 2 is a block diagram illustrating a main configuration of the endoscope system 1 according to the embodiment.

As illustrated in Fig. 1, the electronic endoscope system 1 is a system specialized for medical use, and includes an electronic scope 10 (an example of an electronic endoscope), a processor 20 (an example of a processor for an electronic endoscope), and a monitor 30.

As illustrated in Fig. 1, an insertion portion 120 having flexibility and configured to be inserted into the human body is provided at a distal end of the electronic scope 10. In the vicinity of the distal end of the insertion portion 120, a bending portion 104 that is bent in accordance with a remote operation from a hand operation unit 122 connected to a proximal end of the insertion portion 120 is provided. A bending mechanism of the bending portion 104 is a known mechanism incorporated in a general endoscope. A bending structure bends the bending portion 104 by pulling an operation wire in conjunction with a rotation operation of a bending operation knob provided in the hand operation unit 122. A distal end portion 102 including a solid-state image sensor (hereinafter, referred to as an image sensor)14 (see Fig. 2) is connected to a distal end of the bending portion 104. When the direction of the distal end portion 102 is changed according to the bending operation of the bending portion 104 by the rotation operation of the bending operation knob, an imaging region by the electronic scope 10 is moved.

A light carrying bundle (LCB) 11 (see Fig. 2) is arranged over substantially the entire length from a connector unit 10c to the distal end portion 102 of the electronic scope 10. The LCB 11 is an optical fiber bundle, and guides irradiation light supplied from a light source device 25 to the distal end portion 102 of the electronic scope 10.

The processor 20 is a device that performs signal processing on a video signal of a captured image of an object obtained by capturing the object by the image sensor 14 of the electronic scope 10, and supplies the video signal to the monitor 30 (see Fig. 2).

As illustrated in Fig. 1, the processor 20 is provided with the connector unit 20c for connection with the electronic scope 10. A connector unit 10c for connection with the connector unit 20c of the processor 20 is provided at a proximal end of the electronic scope 10. When the connector unit 10c and the connector unit 20c are mechanically connected, the electronic scope 10 and the processor 20 are electrically connected, and the light source device 25 and the electronic scope 10 are optically connected.

An information processing device 40 is a device, such as a computer device, a tablet terminal, or a smartphone, capable of performing predetermined arithmetic processing, and can be electrically connected to the processor 20 via a cable 200.

The information processing device 40 calculates a parameter for image correction for the electronic scope 10 and/or the processor 20, and records the calculated parameter in the electronic scope 10 and/or the processor 20. In the example illustrated in Fig. 1, the information processing device 40 is not directly connected to the electronic scope 10 by wire, but in a case where the electronic scope 10 and the processor 20 are electrically connected, the parameter can be written in the electronic scope 10 via the processor 20. Note that, as long as the information processing device 40 can wirelessly communicate with the electronic scope 10 and with the processor 20, the information processing device 40 may write respective parameters into the electronic scope 10 and the processor 20 without using the cable 200.

In the embodiment, as described below, the information processing device 40 is configured to calculate a processor matrix and a scope matrix as parameters, write the processor matrix into a matrix storage unit 28 of the processor 20, and write the scope matrix into a matrix storage unit 16 of the electronic scope 10.

The timing of calculating and writing the processor matrix and/or the scope matrix is not limited, but for example, a timing after manufacturing but before shipping of the electronic scope 10 and the processor 20 is preferable.

Referring to Fig. 2, the processor 20 includes a system controller 21, an image input processing unit 22, an image memory 23, an image output processing unit 24, the light source device 25, an operation panel 26, a color correction calculation unit 27, the matrix storage unit 28, and a condenser lens 29.

The electronic scope 10 includes the LCB 11, a light distribution lens 12, an objective lens 13, the image sensor 14, a driver signal processing circuit 15, and the matrix storage unit 16.

The system controller 21 executes various programs and integrally controls the entire electronic endoscope system 1. In addition, the system controller 21 is connected to the operation panel 26. The system controller 21 changes operations of the electronic endoscope system 1 and a parameter for each of the operations in accordance with an operator's instruction input to the operation panel 26. The system controller 21 outputs a clock pulse for adjusting operation timing of each unit to each circuit in the electronic endoscope system 1.

The light source device 25 emits illumination light L for illuminating an object such as a living tissue in a body cavity. The illumination light L includes white light, pseudo white light, or special light. According to the embodiment, it is preferable that the light source device 25 select one of a mode of constantly emitting the white light or the pseudo white light as the illumination light L and a mode of alternately emitting the white light or pseudo white light and the special light as the illumination light L, and emit the white light, the pseudo white light, or the special light based on the selected mode. The white light is light having a flat spectral intensity distribution in a visible light band, and the pseudo white light is light which is a mixture of light of a plurality of wavelength bands and has non-flat spectral intensity distribution. The special light is light of a narrow wavelength band, such as blue or green, in the visible light band. The light of the blue or green wavelength band is used at the time of enhancing a specific portion of the living tissue and observing the portion. The illumination light L emitted from the light source device 25 is condensed onto an incident end face of the LCB 11 by the condenser lens 29, and is incident into the LCB 11 of the electronic scope 10.

Note that, in the example illustrated in Fig. 2, the light source device 25 is built in the processor 20, but the light source device 25 may be configured separately from the processor 20.

The illumination light L incident into the LCB 11 propagates through the LCB 11 of the electronic scope 10. The illumination light L propagating through the LCB 11 is emitted from an exit end face of the LCB 11 arranged at the distal end portion 102 of the electronic scope 10, and is applied to the object via the light distribution lens 12. Return light from the object illuminated with the illumination light L from the light distribution lens 12 forms an optical image on a light receiving surface of the image sensor 14 via the objective lens 13.

The image sensor 14 is a single-plate color charge coupled device (CCD) image sensor having a Bayer pixel arrangement. The image sensor 14 accumulates the optical image formed by each of pixels on the light receiving surface, as charge corresponding to the amount of light, and generates and outputs image signals of red (R), green (G), and blue (B). Note that the image sensor 14 is not limited to a CCD image sensor, and may be replaced with a complementary metal oxide semiconductor (CMOS) image sensor or other types of imaging devices. Furthermore, the image sensor 14 may include a complementary color filter.

A clock pulse is supplied from the system controller 21 to the driver signal processing circuit 15 of the electronic scope 10. The driver signal processing circuit 15 performs driving control for the image sensor 14 at a timing synchronized with the frame rate of a video image processed on the processor 20 side in accordance with the clock pulse supplied from the system controller 21. For example, the frame rate is 1/30 seconds. The driver signal processing circuit 15 performs predetermined processing including A/D conversion on the image signal input from the image sensor 14 and outputs the processed image signal to the image input processing unit 22 of the processor 20.

The image input processing unit 22 performs predetermined signal processing such as demosaic processing and matrix calculation on the input image signal. The image input processing unit 22 may perform predetermined noise reduction processing.

The image memory 23 is a memory for buffering the image signal (image) processed by the image input processing unit 22.

The image output processing unit 24 sequentially processes the images in the image memory 23 to generate monitor display screen data, and converts the generated monitor display screen data into a predetermined video format signal. The converted video format signal is output to the monitor 30. With this processing, an image of the object is displayed on a display screen of the monitor 30.

The matrix storage unit 28 of the processor 20 and the matrix storage unit 16 of the electronic scope 10 are non-volatile memories such as a solid state drive (SSD), for example, and store a processor matrix and a scope matrix, respectively. As described above, the processor matrix and the scope matrix are written by the information processing device 40.

The color correction calculation unit 27 of the processor 20 performs color correction calculation on an image buffered in the image memory 23 based on the processor matrix and the scope matrix. The system controller 21 overwrites the image memory 23 with the image calculated by the color correction calculation unit 27. Therefore, the image data for monitor display generated by the image output processing unit 24 is based on the image subjected to the color correction.

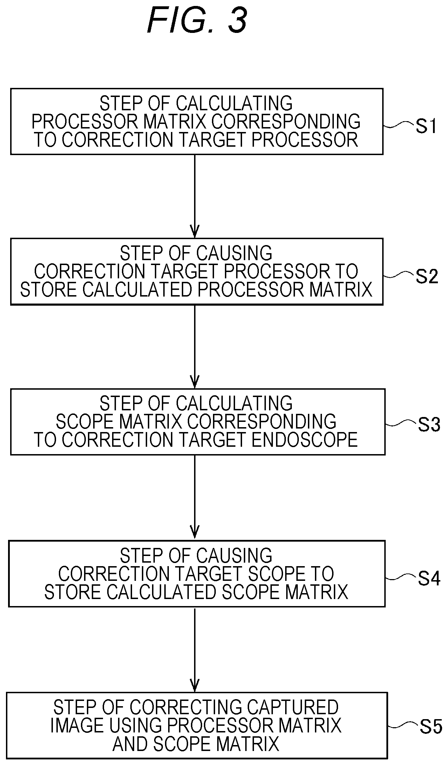

Next, the image correction method in the electronic endoscope system according to the embodiment will be described with reference to Figs. 3 to 6.

Fig. 3 is a diagram indicating each step of the image correction method in the electronic endoscope system according to the embodiment. As indicated in Fig. 3, the image correction method includes steps S1 to S5.

Steps S1 and S2 are steps performed on the processor to be corrected, and are performed, for example, at the time after manufacturing but before shipping of the processor.

Steps S3 and S4 are steps performed on the electronic scope to be corrected, and are performed, for example, at the time after manufacturing but before shipping of the electronic scope.

Step S5 is a step performed on each image in units of frames at the time of diagnosis.

### (Step S1)

Step S1 is a step of calculating a processor matrix (an example of a first correction parameter) corresponding to a correction target processor 20_Tar which is a processor to be corrected, using a reference electronic scope 10_Ref which is an electronic scope serving as a reference for correction and a reference processor 20_Ref which is a processor serving as a reference for correction.

The reference electronic scope 10_Ref is an electronic scope 10 serving as a master to be a target of color correction. For example, in general, image sensors provided in electronic scopes have an individual difference in spectral characteristics, but the reference electronic scope 10_Ref is an electronic scope 10 provided with an image sensor 14 in which the spectral characteristic is the median value.

The reference processor 20_Ref is a processor 20 serving as a master to be a target of color correction. For example, in general, illumination light of light source devices provided in processors have an individual difference in spectral characteristics, but the reference processor 20_Ref is a processor 20 provided with a light source device 25 in which the spectral characteristic of the illumination light is the median value.

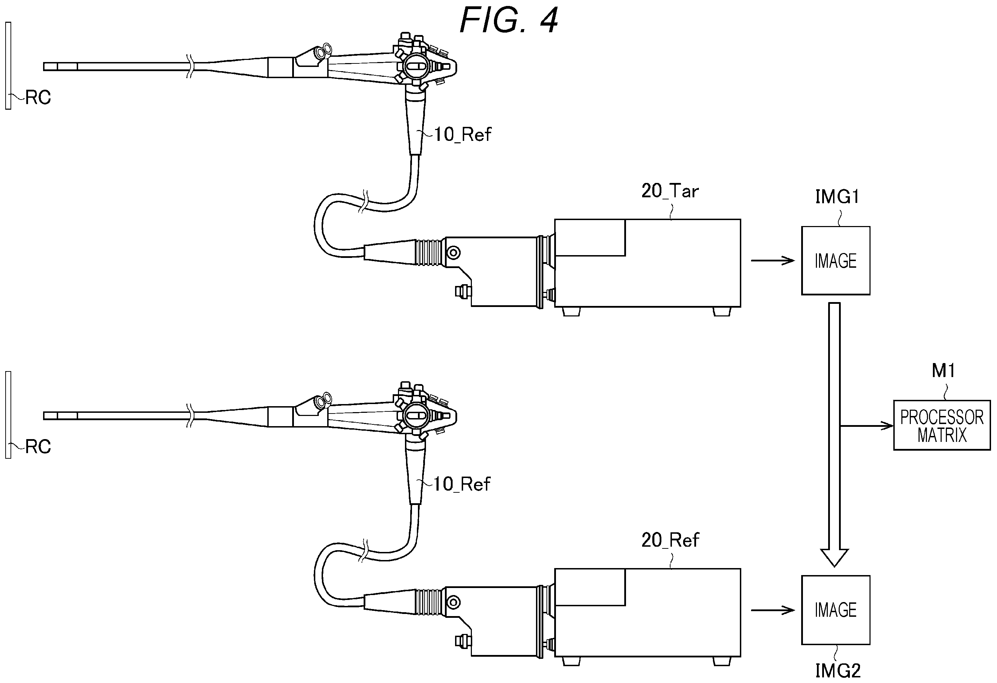

Fig. 4 conceptually illustrates a procedure in step S1.

First, the reference electronic scope 10_Ref and the correction target processor 20_Tar are connected and then, the information processing device 40 not illustrated in Fig. 4 is connected to the correction target processor 20_Tar. A distal end portion of the reference electronic scope 10_Ref is directed to a color index RC serving as a reference. The color index RC is an index in which a color serving as a reference at the time of correction is displayed. The color displayed on the color index RC is appropriately determined according to the object, but in a case where the living tissue is imaged, it is preferable that the color have strong redness in accordance with the color of the affected part. A plurality of color indexes RC including different colors may be used.

In step S1, a captured image IMG1 of the color index RC obtained by the combination of the reference electronic scope 10_Ref and the correction target processor 20_Tar is acquired into the information processing device 40.

Next, similarly, a captured image IMG2 of the color index RC obtained by the combination of the reference electronic scope 10_Ref and the reference processor 20_Ref is acquired into the information processing device 40.

The information processing device 40 calculates a processor matrix M1 for matching the captured image IMG1 with the captured image IMG2. At the time of calculating the processor matrix M1, a matrix for converting a pixel value of a specific pixel in the captured image IMG1 (RGB value; an example of a first pixel value) into a pixel value of a corresponding pixel in the captured image IMG2 (an example of a second pixel value) may be obtained, or a matrix for converting an average pixel value (an average value of RGB) of pixels within the corresponding range of each captured image may be obtained.

### (Step S2)

Step S2 is a step of causing the matrix storage unit 28 of the correction target processor 20_Tar to store the processor matrix calculated in step S1.

In step S2, the information processing device 40 transmits the processor matrix M1 calculated in step S1 to the correction target processor 20_Tar.

Steps S1 and S2 are typically performed at a manufacturing site of the processor 20 before shipping of the processor 20. That is, at least one reference electronic scope 10_Ref and at least one reference processor 20_Ref are prepared at the manufacturing site of the processor 20, the plurality of processors 20 to be produced are sequentially set as the correction target processor 20_Tar one by one, and the processor matrix M1 is individually calculated and stored for each processor 20. Note that, in Fig. 4, the acquisition of the captured image IMG2 by a combination of the reference electronic scope 10_Ref and the reference processor 20_Ref is required once, and is not required to be performed sequentially.

### (Step S3)

Step S3 is a step of calculating a scope matrix (an example of a second correction parameter) corresponding to a correction target scope 10_Tar which is an electronic scope to be corrected, using the reference electronic scope 10_Ref and the reference processor 20_Ref.

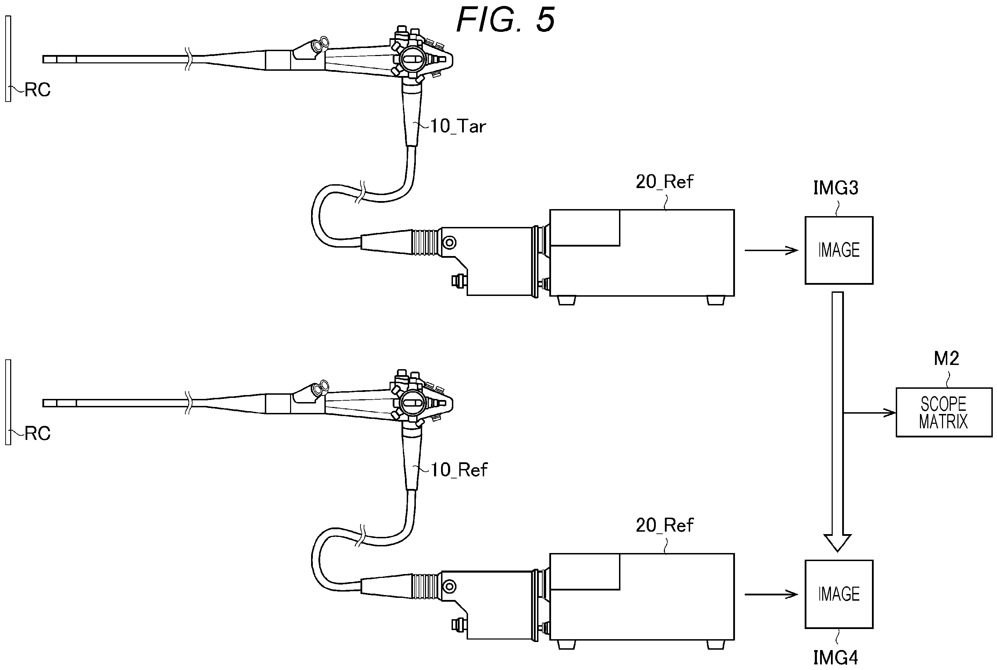

Fig. 5 conceptually illustrates a procedure in step S3.

First, the correction target scope 10_Tar and the reference processor 20_Ref are connected and then, the information processing device 40 not illustrated in Fig. 5 is connected to the reference processor 20_Ref. A distal end portion of the correction target scope 10_Tar is directed to the color index RC serving as a reference.

In step S3, a captured image IMG3 of the color index RC obtained by the combination of the correction target scope 10_Tar and the reference processor 20_Ref is acquired into the information processing device 40.

Then, similarly, a captured image IMG4 of the color index RC obtained by the combination of the reference electronic scope 10_Ref and the reference processor 20_Ref is acquired into the information processing device 40.

The information processing device 40 calculates a scope matrix M2 for matching the captured image IMG3 with the captured image IMG4. At the time of calculating the scope matrix M2, a matrix for converting a pixel value of a specific pixel in the captured image IMG3 (an example of a third pixel value) into a pixel value of a corresponding pixel in the captured image IMG4 (an example of a fourth pixel value) may be obtained, or a matrix for converting an average pixel value (an average value of RGB) of pixels within the corresponding range of each captured image may be obtained.

### (Step S4)

Step S4 is a step of causing the matrix storage unit 16 of the correction target scope 10_Tar to store the scope matrix calculated in step S3.

In step S4, the information processing device 40 transmits the scope matrix M2 calculated in step S3 to the correction target scope 10_Tar via the reference processor 20_Ref.

Steps S3 and S4 are typically performed at a manufacturing site of the electronic scope 10 before shipping of the electronic scope 10. That is, at least one reference electronic scope 10_Ref and at least one reference processor 20_Ref are prepared at the manufacturing site of the electronic scope 10, the plurality of electronic scopes 10 to be produced are sequentially set as the correction target scope 10_Tar one by one, and the scope matrix M2 is individually calculated and stored for each electronic scope 10. Note that, in Fig. 5, the acquisition of the captured image IMG4 by a combination of the reference electronic scope 10_Ref and the reference processor 20_Ref is required once, and is not required to be performed sequentially.

The reference electronic scope 10_Ref and the reference processor 20_Ref used in steps S1 and S2, and the reference electronic scope 10_Ref and the reference processor 20_Ref used in steps S3 and S4 do not have to be the same. In a case where steps S1 and S2 are performed at the manufacturing site of the processor 20 and steps S3 and S4 are performed at the manufacturing site of the electronic scope 10, it is advantageous to use different reference devices (reference electronic scope 10_Ref and reference processor 20_Ref). Even in that case, as long as the common color index RC is used, there is almost no variation in the calculated processor matrix M1 and scope matrix M2.

### (Step S5)

Step S5 is a step of performing, by the correction target processor 20_Tar, correction on the captured image captured by the image sensor 14 of the correction target scope 10_Tar using the processor matrix M1 and the scope matrix M2 when the correction target scope 10_Tar and the correction target processor 20_Tar are connected.

Unlike steps S1 to S4, step S5 is executed when diagnosis is performed at a medical site using the electronic endoscope system 1 in which the processor 20 storing the processor matrix M1 and the electronic scope 10 storing the scope matrix M2 are connected.

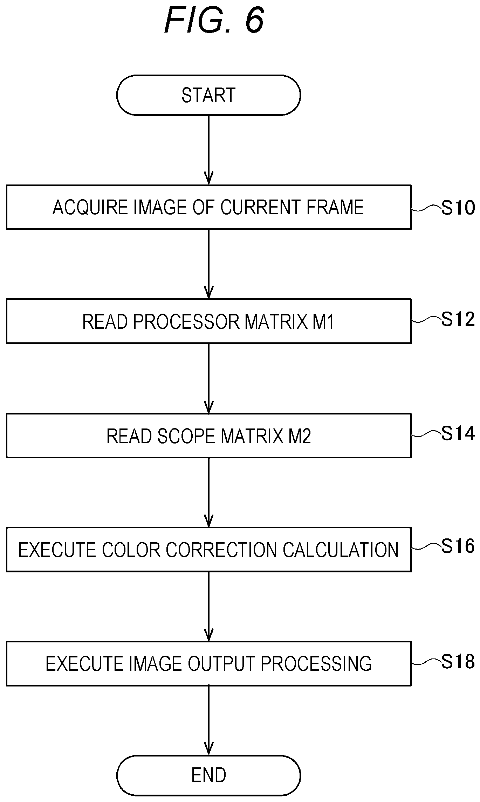

A flowchart of processing executed by the processor 20 in step S5 is indicated in Fig. 6.

First, the processor 20 acquires an image of the current frame from the electronic scope 10, performs predetermined image processing, and temporarily stores the image in the image memory 23 (step S10). The processor 20 reads the processor matrix M1 from the matrix storage unit 28 (step S12), and reads the scope matrix M2 from the matrix storage unit 16 of the electronic scope 10 (step S14).

Then, the processor 20 performs a color correction calculation on the image stored in the image memory 23 in step S10 (step S16). Specifically, the processor 20 obtains a pixel value obtained by converting the pixel value of each pixel of the image by a matrix (M1 · M2) which is a product of the processor matrix M1 and the scope matrix M2. The processor 20 executes image output processing on the image obtained by converting the pixel value of each pixel (step S18). In the image output processing, a screen data image for monitor display is generated on the basis of the image, and the generated screen data for monitor display is converted into a predetermined video format signal and output to the monitor 30.

As described above, in the image correction method in the electronic endoscope system according to the embodiment, the scope matrix and the processor matrix optimized in advance are stored before diagnosis in the electronic scope 10 and the processor 20 to be combined, respectively, and the color information can be corrected using the scope matrix and the processor matrix at the time of diagnosis. Therefore, the processing load when correcting the color information as in the conventional system is greatly reduced. The electronic scope 10 and the processor 20 only need to store the scope matrix and the processor matrix, respectively, and also need a small memory capacity.

In the image correction method in the electronic endoscope system described above, since the processor matrix and the scope matrix are determined in a state in which the spectral reflectance characteristic of the color index RC according to the object is reflected, the pixel value (R value, G value, and B value) of the image of each frame can be corrected according to the characteristic of the object. For example, in a case where each matrix is determined on the basis of the captured image of the color index RC in which a color with strong redness corresponding to the color of the affected area is displayed, correction according to the spectral reflectance characteristic of the object (affected area of the patient) can be performed at the time of diagnosis.

In the image correction method in the electronic endoscope system described above, since the processor matrix and the scope matrix optimized in advance are stored in the electronic scope and the processor, it is not necessary to consider a combination of the electronic scope and the processor at the time of diagnosis. That is, even if arbitrary electronic scope and processor are combined at the time of diagnosis, it is possible to perform optimum correction on the captured image.

### (2) Second Embodiment

Hereinafter, a second embodiment will be described.

The second embodiment is different from the first embodiment in that a processor matrix and a scope matrix are calculated in a form including not only an image but also a feature amount of the image, and image correction is performed on the basis of such processor matrix and scope matrix.

Hereinafter, a description will be given focusing on differences from the image correction method (Fig. 5) described in the first embodiment. Steps S1 to S5 of an image correction method according to the present embodiment are as follows.

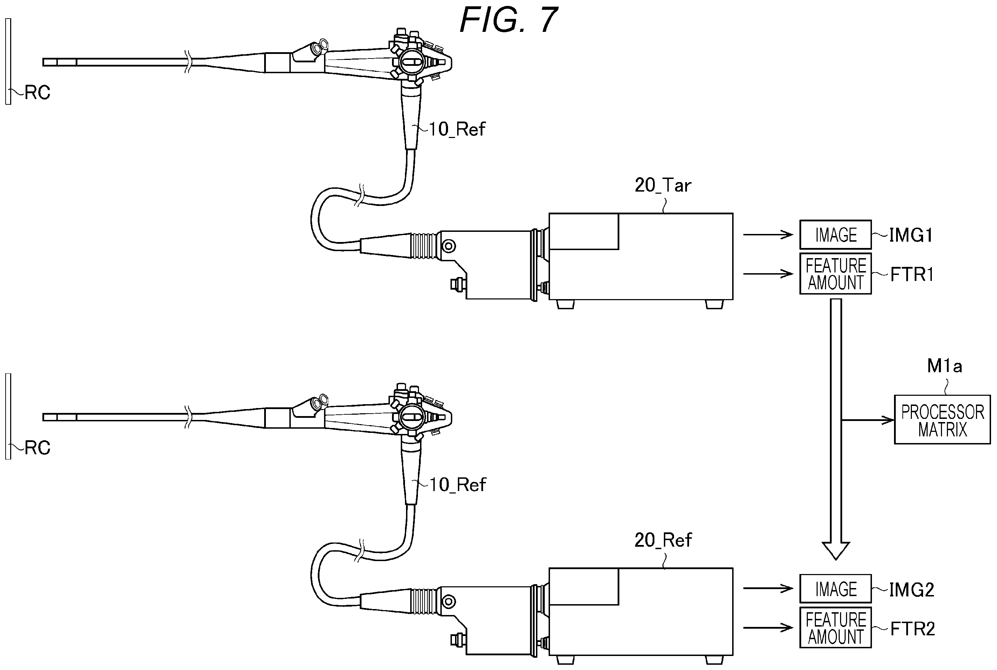

Fig. 7 conceptually illustrates a procedure of calculating, in step S1, a processor matrix M1a including the feature amount with respect to Fig. 4.

Referring to Fig. 7, in step S1, a correction target processor 20_Tar specifies a feature amount FTR1 (an example of a first feature amount) from a pixel value (an example of a first pixel value) of a specific pixel in a captured image IMG1 of a color index RC obtained by the combination of a reference electronic scope 10_Ref and the correction target processor 20_Tar.

A reference processor 20_Ref specifies a feature amount FTR2 (an example of a second feature amount) from a pixel value (an example of a second pixel value) of a corresponding pixel in a captured image IMG2 of the color index RC obtained by the combination of the reference electronic scope 10_Ref and a reference processor 20_Ref.

Then, the information processing device 40 calculates the processor matrix M1a including, in addition to a parameter for converting the captured image IMG1 into the captured image IMG2, a parameter (an example of a first feature amount correction parameter) for converting the feature amount FTR1 into the feature amount FTR2.

In step S2, the processor matrix M1a is stored in the correction target processor 20_Tar.

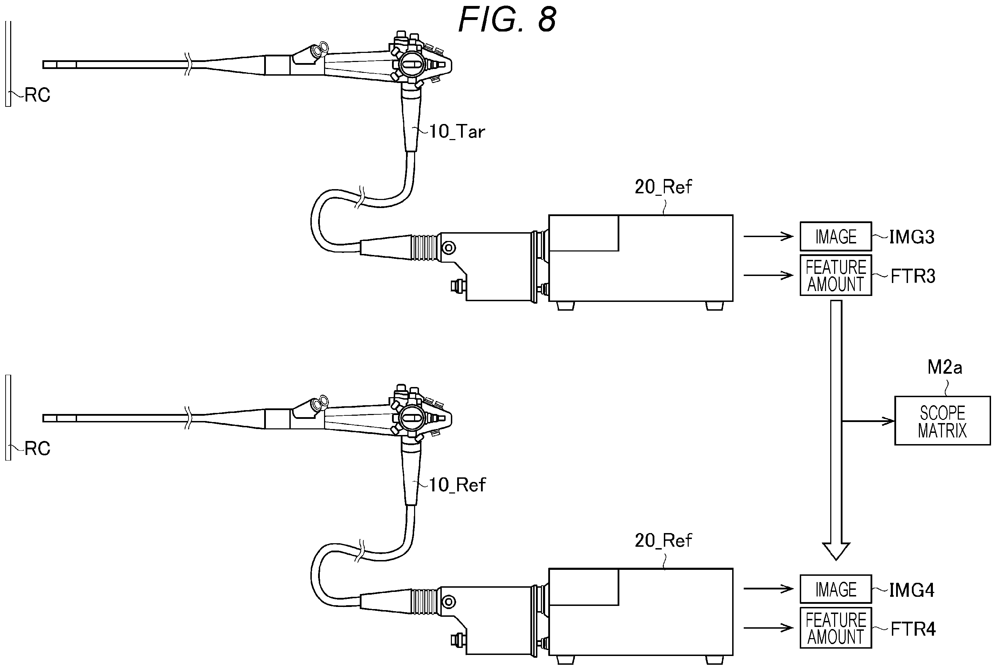

Fig. 8 conceptually illustrates a procedure of calculating, in step S3, a scope matrix M2a including the feature amount with respect to Fig. 5.

Referring to Fig. 8, in step S3, a reference processor 20_Ref specifies a feature amount FTR3 (an example of a third feature amount) from a pixel value (an example of a third pixel value) of a specific pixel of a captured image IMG3 of a color index RC obtained by the combination of a correction target scope 10_Tar and the reference processor 20_Ref.

The reference processor 20_Ref specifies a feature amount FTR4 (an example of a fourth feature amount) from a pixel value (an example of a fourth pixel value) of a corresponding pixel in a captured image IMG4 of the color index RC obtained by the combination of the reference electronic scope 10_Ref and the reference processor 20_Ref.

Then, the information processing device 40 calculates the scope matrix M2a including, in addition to a parameter for converting the captured image IMG3 into the captured image IMG4, a parameter (an example of a second feature amount correction parameter) for converting the feature amount FTR3 into the feature amount FTR4.

In step S4, the scope matrix M2a is stored in the correction target scope 10_Tar.

In step S5, by the correction target processor 20_Tar, correction on the captured image captured by an image sensor 14 of the correction target scope 10_Tar using the processor matrix M1a and the scope matrix M2a is performed when the correction target scope 10_Tar and the correction target processor 20_Tar are connected. Accordingly, the correction target processor 20_Tar also performs correction on the feature amount obtained from the pixel value of the captured image captured by the image sensor 14 of the correction target scope 10_Tar.

The method of calculating the feature amounts in steps S1 and S3 can be appropriately defined. For example, as a value indicating the degree of inflammation at an inflammation site of a patient who is an object, a ratio of pixel values between corresponding pixels (for example, the ratio between the G value and the R value) can be used as the feature amount.

In the present embodiment, since the processor matrix and the scope matrix each include the parameter for correcting the feature amount, it is possible to easily correct the feature amount by executing the color correction calculation on the image of each frame obtained at the time of diagnosis.

Note that, in the present embodiment, it is preferable to determine the color index RC used in steps S1 and S3 from among the plurality of color indexes according to the feature amount obtained from the pixel value of the captured image. By selecting the color index so that the hue of the object is clearly reflected by the feature amount of the captured image, the calculated processor matrix M1a and scope matrix M2a can be set to more appropriate values in correcting the feature amount. For example, as described above, in a case where the ratio between the G value and the R value of the pixel is set as the feature amount, it is preferable to select a color index in which red or a color including red is displayed from a plurality of color indexes so that the degree of redness of the object is clearly reflected by the feature amount.

The present invention relates to a patent application of Japanese Patent Application No. 2022-120393 filed with the Japan Patent Office on July 28, 2022, the entire contents of which are incorporated herein by reference.

## Claims

1. An image correction method of correcting a captured image, in an electronic endoscope system to which an electronic endoscope that captures an image of a living tissue with an image sensor and a processor for an electronic endoscope that performs signal processing on a captured image by the image sensor to create a display image are connected, the image correction method comprising:
a step of calculating a first correction parameter corresponding to a correction target processor which is a processor for an electronic endoscope to be corrected, using a reference endoscope which is an electronic endoscope serving as a reference for correction and a reference processor which is a processor for an electronic endoscope serving as a reference for correction;
a step of causing the correction target processor to store the calculated first correction parameter;
a step of calculating a second correction parameter corresponding to a correction target endoscope which is an electronic endoscope to be corrected, using the reference endoscope and the reference processor;
a step of causing the correction target endoscope to store the calculated second correction parameter; and
a step of performing, by the correction target processor, correction on a captured image captured by an image sensor of the correction target endoscope using the first correction parameter and the second correction parameter when the correction target endoscope and the correction target processor are connected.

2. The image correction method in an electronic endoscope system according to claim 1, wherein
the step of calculating the first correction parameter includes,
based on a first pixel value which is a pixel value of a captured image obtained when the reference endoscope and the correction target processor are connected and a predetermined color index is captured, and
based on a second pixel value which is a pixel value of a captured image obtained when the reference endoscope and the reference processor are connected and the color index is captured,
calculating, as the first correction parameter, a parameter for converting the first pixel value into the second pixel value, and
the step of calculating the second correction parameter includes,
based on a third pixel value which is a pixel value of a captured image obtained when the correction target endoscope and the reference processor are connected and a predetermined color index is captured, and
based on a fourth pixel value which is a pixel value of a captured image obtained when the reference endoscope and the reference processor are connected and the color index is captured,
calculating, as the second correction parameter, a parameter for converting the third pixel value into the fourth pixel value.

3. The image correction method in an electronic endoscope system according to claim 2, further comprising:
a step of specifying a first feature amount which is a feature amount obtained from the first pixel value and a second feature amount which is a feature amount obtained from the second pixel value, and calculating a first feature amount correction parameter for converting the first feature amount into the second feature amount;
a step of specifying a third feature amount which is a feature amount obtained from the third pixel value and a fourth feature amount which is a feature amount obtained from the fourth pixel value, and calculating a second feature amount correction parameter for converting the third feature amount into the fourth feature amount; and
a step of performing, by the correction target processor, correction on a feature amount obtained from a pixel value of a captured image captured by an image sensor of the correction target endoscope using the first feature amount correction parameter and the second feature amount correction parameter when the correction target endoscope and the correction target processor are connected.

4. The image correction method in an electronic endoscope system according to claim 3, wherein
the color index is determined from among a plurality of color indexes according to a feature amount obtained from a pixel value of a captured image.
